# EUROPEAN PATENT APPLICATION

(11) **EP 4 712 083 A1**
(43) Date of publication of application: **18.03.2026**
(21) Application number: 23937416.8
(22) Date of filing: 12.05.2023
(51) Int. Cl.: G16B 5/00

(54) **METABOLIC NETWORK PROCESSING SYSTEM AND METABOLIC NETWORK PROCESSING METHOD**

(71) Applicant: Hitachi, Ltd., Tokyo 100-8280 (JP)
(72) Inventor: HOSODA Shion, Tokyo 100-8280 (JP); SATO Miwa, Tokyo 100-8280 (JP); WATANABE Koichi, Tokyo 100-8280 (JP)
(74) Representative: MERH-IP Matias Erny Reichl Hoffmann Patentanwälte PartG mbB
(86) International application number: PCT/JP2023/018013
(87) International publication number: WO 2024/236670

(57) **Abstract**

The present disclosure proposes, as an example, a metabolic network processing technique which is related to a metabolic network processing system for extracting and proposing a modified enzyme that achieves a target yield of a target substance in a metabolic network in order to constantly predict an increase or decrease in a yield of the target substance when a modification of the enzyme is proposed based on a structural sensitivity analysis. A control device executes (i) processing of generating N sensitivity matrices by using a stoichiometric matrix obtained by converting the metabolic network, a basis c of a kernel of the stoichiometric matrix, and a given probability distribution, (ii) processing of calculating, by checking a sign of each matrix element in the N sensitivity matrices and calculating a proportion of the sign in each matrix element, a confidence level of each matrix element, (iii) processing of extracting k matrix elements having high confidence levels among confidence levels for the number of matrix elements in the sensitivity matrix as modified enzymes, and (iv) processing of outputting the extracted k modified enzymes

## Description

### Technical Field

The present disclosure relates to a metabolic network processing system and a metabolic network processing method.

### Background Art

In substance production using biosynthesis, it is important to improve a yield of a target substance by metabolic network design. Here, biosynthesis is a method for producing a substance through biological activity of microorganisms. Biosynthesis has a lower environmental load than chemical synthesis and can achieve carbon-neutral synthesis. In addition, biosynthesis has been reported to be superior to chemical synthesis in terms of cost.

The metabolic network means a set of enzyme reactions of an organism. The metabolic network design refers to designing a metabolic network suitable for the purpose of substance production or the like by deletion, insertion, or modification of the enzymes. Here, deletion, insertion, and modification of an enzyme respectively refer to removal of an enzyme reaction from a metabolic network, addition of an enzyme reaction to a metabolic network, and change of conditions of an enzyme reaction by genetic recombination, genome editing, plasmid introduction, or the like for an organism. Therefore, the efficiency of substance production can be improved by the metabolic network design. However, it is difficult to select an enzyme to be deleted, inserted, or modified. Therefore, a technique of proposing, based on information on a metabolic network or the like, an enzyme to be deleted, inserted, or modified is important. Among them, modification of an enzyme can utilize a technique of protein engineering, and therefore, it can be said that proposal of a modified enzyme is particularly important.

In order to solve such difficulty in designing a metabolic network, for example, PTL 1 discloses a technique of proposing, using flux balance analysis (hereinafter, referred to as FBA), an enzyme to be deleted and inserted. It should be noted that many empirical examples of deletion and insertion from and into a metabolic network have also been reported.

### Citation List

### Patent Literature

PTL 1: JP2021-16325A

### Non Patent Literature

NPL 1: Fiedler, Bemold, and Atsushi Mochizuki. "Sensitivity of chemical reaction networks: a structural approach. 2. Regular monomolecular systems." Mathematical methods in the applied sciences 38.16 (2015): 3519-3537.

### Summary of Invention

### Technical Problem

However, when the FBA is used, deletion and insertion of an enzyme can be proposed, but modification of the enzyme cannot be proposed. This is because it is necessary to introduce reaction kinetics in order to modify the enzyme, but in the FBA, analysis is performed without introducing the reaction kinetics in order to simplify the problem.

In addition, in order to improve the proposal accuracy, it is desirable to verify the proposed result by a biological experiment and give feedback of a result.

However, deletion and insertion of enzymes change the form of the metabolic network, and therefore, the prediction result cannot be compared with data obtained by biological experiments, and it is difficult to improve the following proposal.

On the other hand, as illustrated in NPL 1, structural sensitivity analysis (hereinafter, referred to as SSA) has attracted attention as a technique of proposing modification of an enzyme, which is important in the metabolic network design. According to the structural sensitivity analysis, it is not required to determine a parameter from data or the like, and the influence on substance production can be predicted when the enzyme is modified only from the structure of the metabolic network. Specifically, it can be predicted whether the yield of the target substance increases, decreases, or does not change when the corresponding enzyme reaction is promoted for all the enzymes on the metabolic network. The modification of the enzyme does not change the form of the metabolic network, and therefore, it is possible to verify the prediction result by a biological experiment and to give feedback of the result.

On the other hand, the SSA is indefinite, that is, there is a case where the increase or decrease in yield cannot be predicted. This is because SSA analysis conditions are strict. Therefore, it may be impossible to propose a modified enzyme only by using the SSA with severe analysis conditions.

In view of such a status, the present disclosure proposes a technique capable of constantly predicting an increase or decrease in a yield of a target substance when a modification of an enzyme is proposed based on structural sensitivity analysis.

### Solution to Problem

In order to solve the above problems, the present disclosure proposes, as an example, a metabolic network processing system for extracting and proposing a modified enzyme that achieves a target yield of a target substance in a metabolic network. The metabolic network processing system includes a storage device configured to hold a metabolic network processing program for extracting and outputting the modified enzyme, and a control device configured to extract the modified enzyme by processing the metabolic network in accordance with the metabolic network processing program read from the storage device. The control device is configured to execute processing of generating N sensitivity matrices using a stoichiometric matrix obtained by converting the metabolic network, a basis c of a kernel of the stoichiometric matrix, and a given probability distribution, processing of calculating, by checking a sign of each matrix element in the N sensitivity matrices and calculating a proportion of the sign in each matrix element, a confidence level of each matrix element, processing of extracting k matrix elements having high confidence levels among confidence levels for the number of matrix elements in the sensitivity matrix as modified enzymes, and processing of outputting the extracted k modified enzymes.

Additional features related to the present disclosure will be clarified from the description of the present description and the accompanying drawings. Aspects of the present disclosure may be achieved and implemented using elements, combinations of various elements, the following detailed description, and accompanying claims.

The description of the present description is merely a typical illustration and does not limit the scope of the claims or application examples of the present disclosure in any sense.

### Advantageous Effects of Invention

According to the technique of the present disclosure, an increase or decrease in yield of a target substance can be constantly predicted when a modification of an enzyme is proposed based on a structural sensitivity analysis.

### Brief Description of Drawings

[FIG. 1] FIG. 1 is a diagram illustrating an outline of a hardware structure of a metabolic network processing system 10 according to an embodiment of the present disclosure.
[FIG. 2] FIG. 2 is a diagram illustrating an example of a sequence of processing executed by a metabolic network system according to the embodiment of the present disclosure.
[FIG. 3] FIG. 3 is a diagram illustrating a display example of an information input GUI and an information output GUI in the processing sequence of FIG. 2.
[FIG. 4] FIG. 4 is a flowchart illustrating an outline of metabolic network processing.
[FIG. 5] FIG. 5 is a flowchart illustrating details of structural sensitivity analysis processing (S401 in FIG. 4).
[FIG. 6] FIG. 6 is a diagram illustrating an example of one sensitivity matrix generated from a metabolic network (a simple example) and a probability distribution p.
[FIG. 7] FIG. 7 is a flowchart illustrating details of extraction of a predetermined number (k) of modified enzymes based on confidence levels (S402).
[FIG. 8] FIG. 8 is a flowchart illustrating details of the determination of the effectiveness of modified enzymes based on biological experiment results (S403).
[FIG. 9] FIG. 9 is a flowchart illustrating details of sequential Bayesian estimation processing (S406).
[FIG. 10] FIG. 10 illustrates an example of a probability distribution p updated by fitting a multivariate log-normal distribution to a sample distribution.

### Description of Embodiments

The present embodiment proposes a metabolic network processing technique capable of constantly predicting an increase or decrease in yield of a target substance in a metabolic network by introducing a new concept of a confidence level into a structural sensitivity analysis to relax conditions for the structural sensitivity analysis and extracting and proposing a modified enzyme having a high confidence level. Hereinafter, the present embodiment will be described with reference to the accompanying drawings. It should be noted that, in the accompanying drawings, elements having the same function may be denoted by the same reference numerals. The accompanying drawings show specific embodiments and implementation examples according to the principle of the present disclosure, but the embodiments and the implementation examples are provided for understanding the present disclosure and are not by no means to be used to limit the present disclosure.

Embodiments are described in sufficient details for those skilled in the art to implement the present disclosure, but it should be understood that other implementations and aspects are possible, and changes in configurations and structures and replacement of various elements are possible without departing from the scope and spirit of the technical idea of the present disclosure. Therefore, the following description is not to be construed as being limited thereto.

### <Description of Terms>

First, the terms used in the present description will be briefly described. The term "enzyme" refers to a biomolecule having a function of converting (catalyzing) a certain substance (compound) into another substance. The term "metabolism" refers to a process of conversion into a substance or energy by a series of enzyme reactions. The term "metabolic network" refers to a network in which a substance generated with another enzyme is changed to another substance with another enzyme, and the transition is expressed as a network. The term "flux (Flux = flow velocity)" refers to a flow of transition of a substance, or a speed or an amount thereof. The flux analysis refers to analysis of the flow, speed, and amount of transition of a substance. The term "flux balance analysis (FBA)" refers to a technique of expressing a flux of a metabolic network by a matrix (stoichiometric matrix) and simulating a transition of conversion of a substance that cannot be visually observed. The term "structure sensitivity analysis (SSA)" is a name of a unique method in which a "structure" is uniquely added to a general word representing the type of analysis called "sensitivity analysis", and refers to a technique of proposing an enzyme that affects an amount of a substance on a metabolic network from the structure (form) of the metabolic network.

The term "modification of an enzyme" refers to changing a parameter that controls a reaction rate of an enzyme. Here, the parameter means a concentration, a rotational speed, substrate affinity, and the like of the enzyme. The term "modified enzyme proposal" refers to a proposal of an enzyme to be modified in order to improve the efficiency of substance production.

Here, the term "biological experiment" refers to an experiment in which an effect of modification of an enzyme in the efficiency of biosynthesis is quantified by comparing an organism in which the enzyme is modified and an organism in which the enzyme is not modified.

The term "probability distribution" refers to the ease of taking a value assumed to be followed by a random variable required for an algorithm of the modified enzyme proposal. The type of the probability distribution is not particularly limited as long as it is not required to be limited by the algorithm. In the present embodiment, for example, a log-normal distribution, a mixed log-normal distribution, a gamma distribution, or the like can be used as the probability distribution.

The term "confidence level" refers to a value obtained by taking an expected value by a probability distribution with respect to an algebraic expression of sensitivity in the structural sensitivity analysis.

The term "sequential Bayesian estimation" refers to updating the probability distribution to a posterior distribution based on Bayesian estimation. An algorithm thereof is not particularly limited. For example, the distribution may be fitted using sample statistics obtained from the posterior distribution by the Markov chain Monte Carlo method, or the sample itself may be used as the distribution for the next modified enzyme proposal.

### <Example of Hardware Structure of Metabolic Network Processing System>

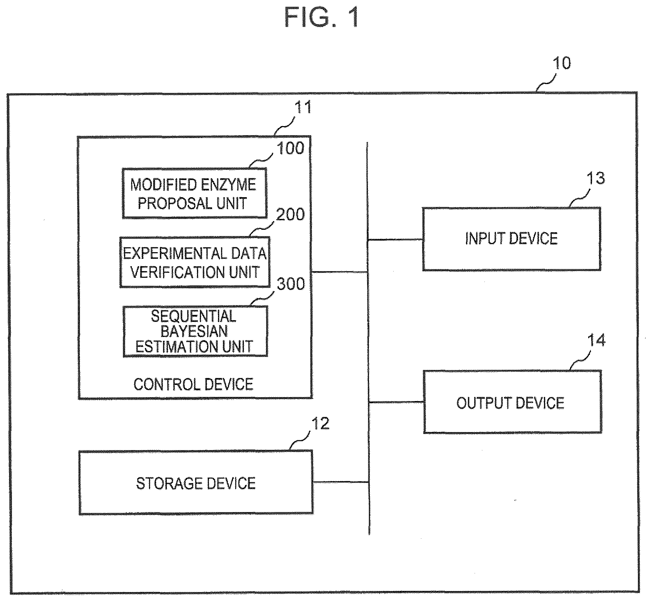

FIG. 1 is a diagram illustrating an outline of a hardware structure of a metabolic network processing system 10 according to the present embodiment. The metabolic network processing system 10 can be implemented by a computer system, and includes, for example, a control device (processor) 11, a storage device 12, an input device 13, and an output device 14.

The control device 11 reads a metabolic network processing program from the storage device 12 and loads the metabolic network processing program in an internal memory (not illustrated) to implement a modified enzyme proposal unit 100, an experimental data verification unit 200, and a sequential Bayesian estimation unit 300.

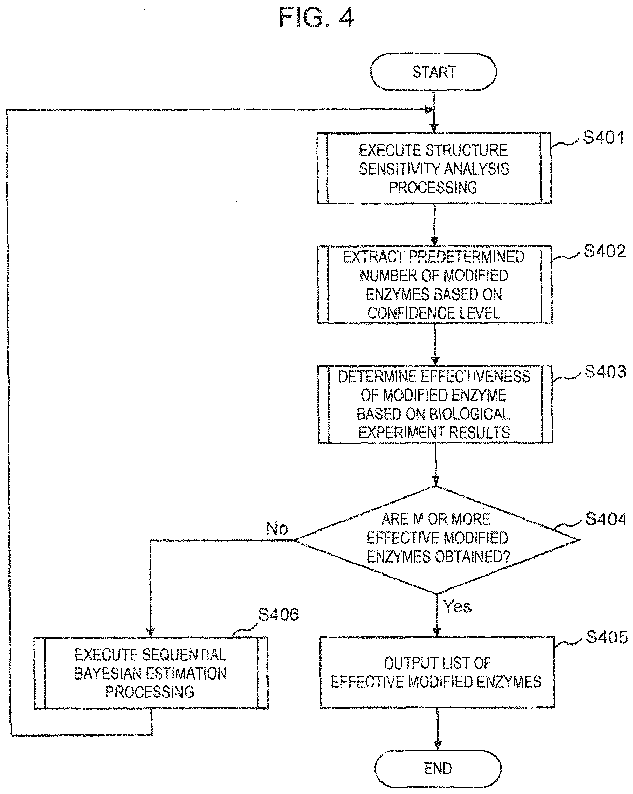

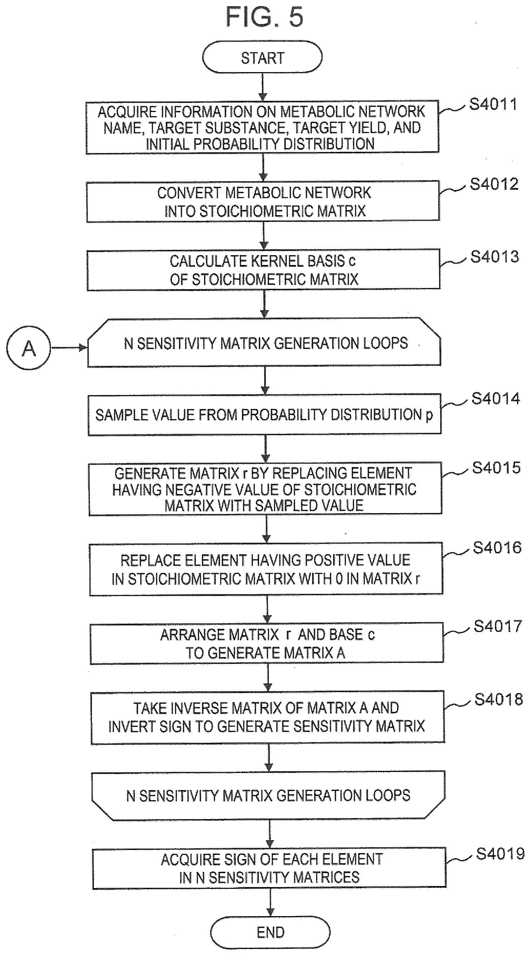

The storage device 12 identifies a modified enzyme for increasing the yield of a target substance in a designated metabolic network based on information and a probability distribution of the metabolic network, and holds a metabolic network processing program for proposing the modified enzyme. The metabolic network processing program is a program corresponding to the flowchart of FIG. 4 (including the flowcharts of FIGS. 5 and 7 to 9) to be described below.

The storage device 12 may be configured to function as a metabolic information database (DB) that stores metabolic networks corresponding to metabolic network names and identification information. When the storage device 12 does not include the metabolic information DB, the control device 11 may access an external database (for example, a public database such as KEGG or Metacyc) via a communication network (not illustrated) to obtain a metabolic network designated by a user.

The input device 13 is implemented by, for example, a keyboard, a mouse, a touch panel, and a switch. For example, the input device 13 can be used when an operator (user) inputs (designates) information (name and identification information) on a metabolic network as a processing target, target substance information (information on a substance whose yield is desired to be improved), target yield information (target value of the yield), information (probability distribution name and identification information) of a probability distribution used as an initial value, and the like to the metabolic network processing system 10 via, for example, a GUI.

The output device 14 is implemented by a display device, a printer, or the like, and displays, for example, the above GUI, a result obtained by processing the metabolic network (for example, proposal of a modified enzyme), and an experimental result to be described below (for example, information on a yield obtained by an organism) on a display screen.

In FIG. 1, as the computer system, the control device 11, the storage device 12, the input device 13, and the output device 14 are connected via a data line/control line, but the metabolic network processing system 10 may be configured such that the devices are remotely disposed and connected via a communication network.

### <Example of Processing Sequence>

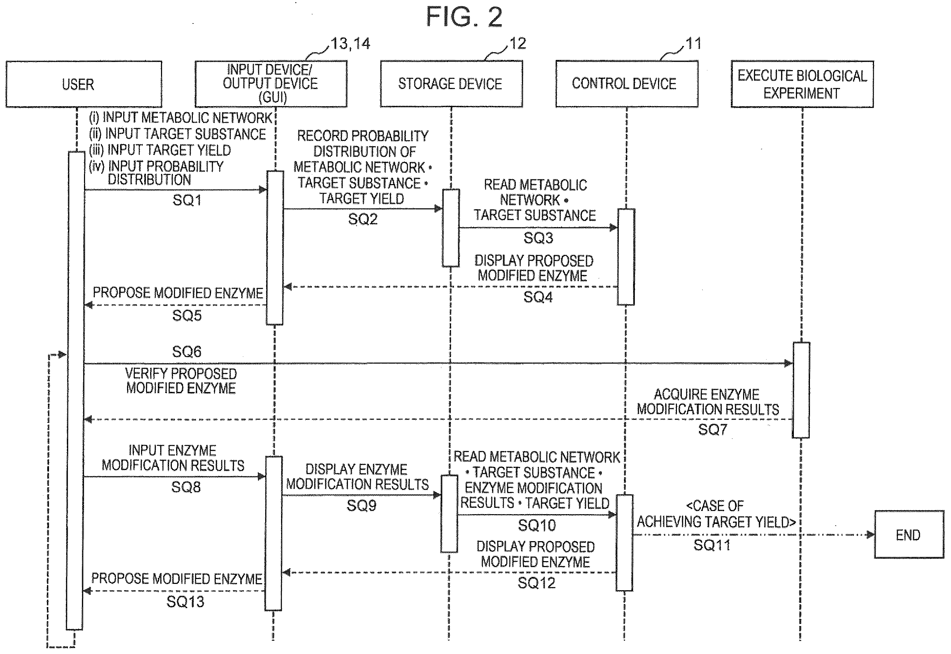

FIG. 2 is a diagram illustrating an example of a sequence of processing executed by a metabolic network system according to the present embodiment.

First, the user operates the input device 13 to input or designate (i) the metabolic network information, (ii) the target substance information, (iii) the target yield information, (iv) the probability distribution information, and the like to an information input graphical user interface (GUI) (SQ1). Then, the control device 11 stores the input or designated pieces of information in the storage device 12 (SQ2). The control device 11 reads the metabolic network and the target substance input (designated) by the user from the storage device 12 (SQ3). As described above, the information may be acquired from an external DB. Then, in accordance with the metabolic network processing program, the control device 11 executes the structural sensitivity analysis (analysis in which the degree of strictness of the application condition in the common SSA is lowered) in which the confidence level is introduced, extracts (as candidates) a predetermined number (for example, five) of modified enzymes (modified enzymes with high confidence levels) in a target metabolic network, and displays (proposes) the modified enzymes on an information output GUI on the screen of the output device (display device) 14 (SQ4 and SQ5). Here, the modified enzyme means an enzyme that may achieve an improvement in yield by modification.

In order to verify the proposed modified enzyme (verify whether the target yield is achieved), the user performs a biological experiment (actually modifying the enzyme to check a change in yield) (SQ6). In addition, the user acquires an enzyme modification result found by performing the biological experiment (SQ7), and operates the input device 13 to input the enzyme modification result to the information input GUI.

The control device 11 records, in the storage device 12, the enzyme modification result (experimental result) input by the user (SQ9). Then, the control device 11 reads information on the metabolic network, the target substance, and the target yield initially input (designated) by the user together with the enzyme modification result (experimental result) from the storage device (SQ10). Subsequently, for example, the control device 11 compares data corresponding to the metabolic network, the target substance, and the enzyme modification result (experimental result) in the biological experiment with data corresponding to the metabolic network, the target substance, and the target yield designated by the user. If there are a predetermined number (for example, 10) of modified enzymes that achieve the target yield, the control device 11 ends the processing (SQ11). On the other hand, if the number of modified enzymes that achieve the target yield does not satisfy the predetermined number, the control device 11 executes sequential Bayesian estimation to change (update) the probability distribution. Then, the control device 11 uses the changed probability distribution to execute the structural sensitivity analysis in which the confidence level is introduced, extracts a predetermined number (for example, five) of modified enzymes, and displays (proposes) these modified enzymes as proposed modified enzymes on the output GUI of the output device 14 again (SQ12 and SQ13).

The user performs the biological experiment again to verify the additionally proposed modified enzyme (SQ6). Thereafter, the processing sequence from SQ6 to SQ13 is repeated until a predetermined number (for example, 10) or more of modified enzymes that achieve the target yield are obtained.

### <Example of GUI Display in Processing Sequence>

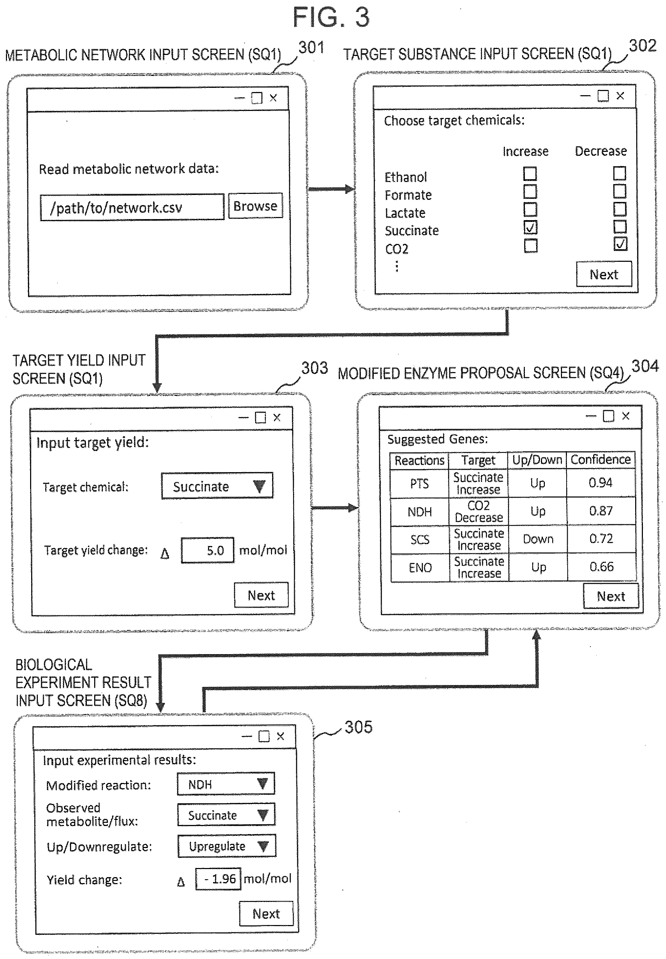

FIG. 3 is a diagram illustrating a display example of an information input GUI and an information output GUI in the processing sequence of FIG. 2.

When the user presses an enzyme modification proposal processing start button (for example, a start button (not illustrated) provided on the information input GUI) by operating the input device 13, the control device 11 displays a metabolic network input screen 301 (corresponding to SQ1), a target substance input screen 302 (SQ1), a target yield input screen 303 (SQ1), and a probability distribution input screen (not illustrated: SQ1) at a timing of information input in response to the pressing of the button (input of an instruction to start enzyme modification proposal processing). The display order is not required to be the order illustrated in FIG. 3. The GUI may be configured such that the metabolic network, the target substance, the target yield, and the probability distribution can be input on one input screen.

For example, the example of FIG. 3 illustrates that the user refers to a file of metabolic network data on the metabolic network input screen 301 and performs selection to propose a modified enzyme for the purpose of increasing a yield of succinic acid (Succinate) and decreasing a yield of carbon dioxide (CO₂) on the target substance input screen 302. On the target yield input screen 303, it is designated that the target yield of succinic acid (Succinate) is increased by 5.0 mol/mol (mol/mol: the amount (amount of succinic acid) per 1 mol of glucose).

When the input of the information required for the modified enzyme proposal processing is completed, the control device 11 executes the structural sensitivity analysis in which the confidence level is introduced, extracts a predetermined number (for example, five) of modified enzymes, and displays these modified enzymes on the modified enzyme proposal screen 304 as modified enzymes to be proposed, as described above (SQ4 and SQ12). In the example of FIG. 3, a proposal list of modified enzymes for increasing succinic acid or decreasing CO₂ is displayed. Although the list is displayed on the modified enzyme proposal screen 304, another display form may be used.

The user performs a biological experiment on the proposed modified enzyme or a modified enzyme separately considered by the user, and verifies a change (increase or decrease) in the yield of the target substance due to the enzyme. Then, the user inputs the experimental result, that is, the numerical value of the increase or decrease in the yield on the biological experiment result input screen 305 (SQ8).

The modified enzyme display on the modified enzyme proposal screen 304 and the input of the experimental result on the biological experiment result input screen 305 are repeated until a desired number (for example, 10) of modified enzymes for achieving the target yield are obtained (SSQ6 to SQ13).

### <Outline of Metabolic Network Processing>

FIG. 4 is a flowchart illustrating an outline of metabolic network processing. The operation subject of each step of the flowcharts illustrated in FIGS. 4 to 7 is the modified enzyme proposal unit 100, the experimental data verification unit 200, or the sequential Bayesian estimation unit 300. These units are implemented by the control device 11 executing the metabolic network processing program, and thus the control device 11 may be the operation subject.

### (i) S401

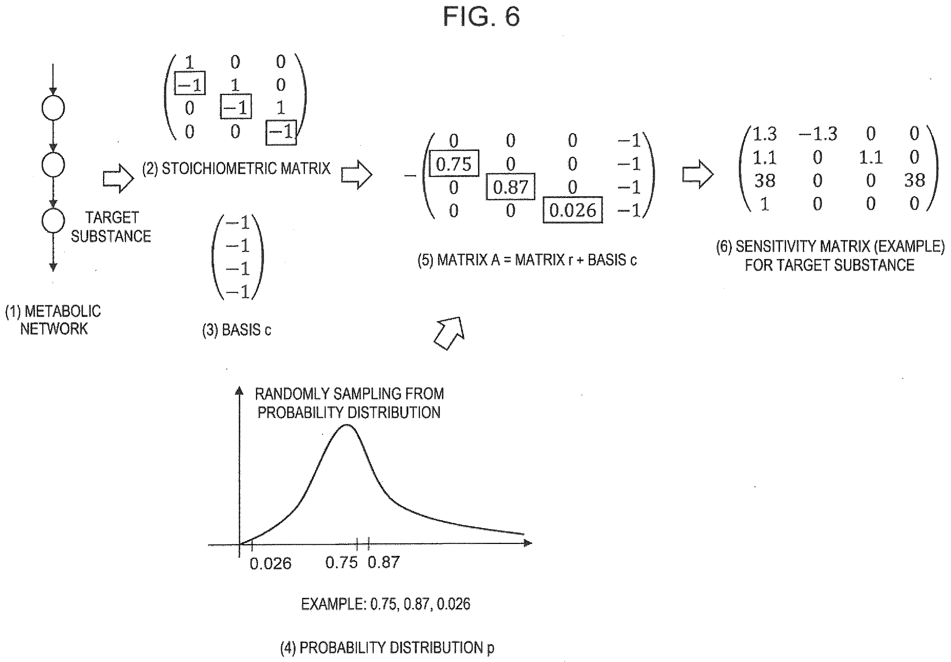

The modified enzyme proposal unit 100 executes structural sensitivity analysis processing using a metabolic network, a target substance, and a probability distribution p designated by the user, and generates N (predetermined number: changeable) sensitivity matrices. Details of the processing of S401 will be described below with reference to FIGS. 5 and 6.

### (ii) S402

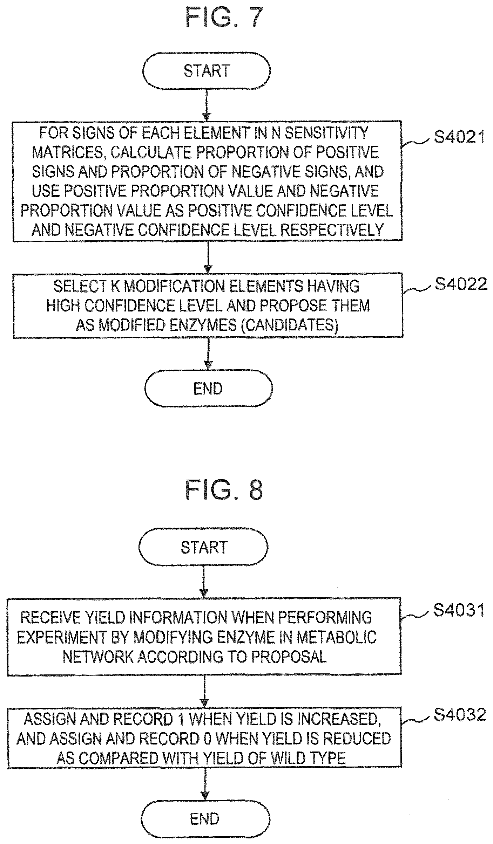

The modified enzyme proposal unit 100 calculates a confidence level based on a sign (positive or negative) of each element in each of the N sensitivity matrices. Here, the confidence level refers to a value obtained by taking an expected value according to a probability distribution for an algebraic expression of sensitivity in the structural sensitivity analysis as described above. Specifically, here, the confidence level is a proportion of positive signs and a proportion of negative signs of each element in the N sensitivity matrices. A positive proportion is a positive confidence level, and a negative proportion is a negative confidence level. Then, the modified enzyme proposal unit 100 selects k (for example, k = 5) enzymes having high confidence levels, and proposes these enzymes as modified enzymes (candidates). For example, when the positive confidence level of a certain element (1, 1) in the sensitivity matrix exceeds 80% and is within k in descending order of the confidence level in the sensitivity matrix group, an enzyme corresponding to the element is proposed as a modified enzyme (candidate). Details of the processing of S402 will be described below with reference to FIG. 7.

When the modified enzyme is proposed, the user actually modifies the target enzyme in a target metabolic network and performs a biological experiment.

### (iii) S403

The experimental data verification unit 200 acquires a result of an actual biological experiment input by the user, and determines the effectiveness of the modified enzyme based on the experimental result. Details of the processing of S403 will be described later with reference to FIG. 8.

### (iv) S404

The modified enzyme proposal unit 100 checks whether M (for example, M = 10) or more effective modified enzymes determined in S403 have been obtained. If M or more effective modified enzymes have been obtained (Yes in S404), the processing proceeds to S405. If M or more effective modified enzymes have not been obtained (No in S404), the processing proceeds to S406.

The reason why a plurality of (M) modified enzymes are proposed is that it is required to cope with a situation in which a desired effect cannot be obtained even when a modification (increase) is performed on a proposed enzyme and another enzyme is desired to be tested, and in addition, when there is a situation in which a predetermined enzyme is not desired to be modified, if a desired purpose can be achieved by modifying another enzyme, a desire to cope with the desired purpose with another enzyme is met.

### (v) S405

The modified enzyme proposal unit 100 creates and outputs (presents to the user) a proposal list (modified enzyme proposal list) including M or more effective modified enzymes obtained in S403.

### (vi) S406

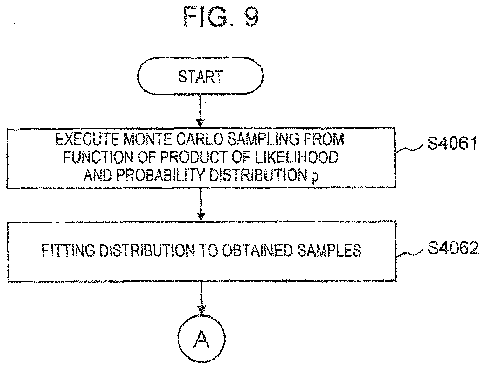

The sequential Bayesian estimation unit 300 subjects the probability distribution p to the sequentially Bayesian estimation using the result obtained in the biological experiment (verification experiment), and generates a new probability distribution p. Details of the processing of S406 will be described below with reference to FIG. 9.

### <Details of Structural Sensitivity Analysis Processing (S401)>

FIG. 5 is a flowchart illustrating details of the structural sensitivity analysis processing (S401 in FIG. 4). FIG. 6 is a diagram illustrating an example of one sensitivity matrix generated from a metabolic network (a simple example) and a probability distribution p.

### (i) S4011

The modified enzyme proposal unit 100 acquires a metabolic network name or metabolic network identification information, information on a target substance whose yield is desired to be changed (increased or decreased), information on a target yield of the target substance, and information on the initial probability distribution, which are respectively input from the GUIs (the metabolic network input screen 301, the target substance input screen 302, the target yield input screen 303, and the probability distribution input screen) by operating the input device 13 by the user. The modified enzyme proposal unit 100 accesses a metabolic network DB (DB installed inside or outside the system) and acquires a metabolic network (detailed information of a structure or the like) based on the acquired metabolic network name and the like. Further, the modified enzyme proposal unit 100 acquires data of the probability distribution p designated by the user from the storage device 12 or another DB.

### (ii) S4012

The modified enzyme proposal unit 100 converts the metabolic network into a stoichiometric matrix representing the metabolic network.

In the example of FIG. 6, a simple (1) metabolic network is illustrated. The metabolic network is converted into (2) a stoichiometric matrix.

### (iii) S4013

The modified enzyme proposal unit 100 calculates (3) a basis c of a kernel from (2) the stoichiometric matrix.

Subsequently, when the processing of S4013 is completed, the modified enzyme proposal unit 100 executes N (for example, 10000) sensitivity matrix generation loops (S4014 to S4018).

### (iv) S4014

The modified enzyme proposal unit 100 performs random sampling on the probability distribution p to acquire a plurality of sample values. The number of samplings matches, for example, the number of elements indicating a negative value in (2) the stoichiometric matrix. It should be noted that values of 0.026, 0.75, and 0.87 are acquired by random sampling from (4) the probability distribution p in FIG. 6.

### (v) S4015

The modified enzyme proposal unit 100 generates a matrix r by replacing a matrix element having a negative value in the stoichiometric matrix with a sampling value acquired by S4014. For example, the matrix r is a matrix generated by replacing three "-1" surrounded by a rectangle in (2) the stoichiometric matrix in FIG. 6 with the sampling values 0.75, 0.87, and 0.026, respectively.

### (vi) S4016

The modified enzyme proposal unit 100 replaces an element corresponding to an element having a positive value among the matrix elements in the stoichiometric matrix with "0" in the matrix r. For example, in FIG. 6, all positive values "1" in the stoichiometric matrix are replaced with "0".

### (vii) S4017

The modified enzyme proposal unit 100 generates a matrix A by arranging the basis c and a matrix r' obtained by replacing a matrix element corresponding to the matrix element having a positive value in the stoichiometric matrix with "0". In FIG. 6, (5) the matrix A is a matrix of 4 rows and 4 columns, which is obtained by arranging the elements of the matrix r (4 × 3 matrix) and the basis c at the 4-th column.

### (viii) S4018

The modified enzyme proposal unit 100 takes an inverse matrix of the matrix A acquired in S4017, and further inverts the sign thereof to generate a sensitivity matrix. FIG. 6 illustrates a state in which (6) one sensitivity matrix is generated from (5) the matrix A.

As described above, one sensitivity matrix is generated by the processing from S4014 to S4018. Then, the sampling processing (S4014) in the probability distribution p is executed N times, and N sensitivity matrices are generated by using new sampling values. When the N sensitivity matrices are generated, the processing proceeds to S4019.

### (ix) S4019

The modified enzyme proposal unit 100 acquires a sign (positive or negative) of each element in the generated N sensitivity matrices. For example, in the case of a 4 × 4 matrix as in (6) the sensitivity matrix in FIG. 6, acquisition (collection) is performed from a sign at a first row and a first column in N (for example, 10000) sensitivity matrices to a sign at a fourth row and a fourth column in N (10000) sensitivity matrices. The processing up to S4019 corresponds to the existing structural sensitivity analysis processing.

### <Details of Extraction of Predetermined Number (k) of Modified Enzymes Based on Confidence Level (S402)>

FIG. 7 is a flowchart illustrating details of extraction of a predetermined number (k) of modified enzymes based on confidence levels (S402). In the present embodiment, unlike the existing structural sensitivity analysis processing, the concept of confidence level is introduced to relax an application condition for an analysis.

### (i) S4021

The modified enzyme proposal unit 100 calculates a proportion of positive signs and a proportion of negative signs for N signs in the elements based on the information on the signs of the elements in the N sensitivity matrices acquired in S4019. Then, the modified enzyme proposal unit 100 defines a positive proportion value as a positive confidence level and a negative proportion value as a negative confidence level. Therefore, (the number of elements in the sensitivity matrix × 2) confidence levels are calculated.

Each element in the sensitivity matrix corresponds to an enzyme to be modified. Therefore, the fact that the confidence level is high in the matrix element means that there is a high possibility that the yield of the target substance is changed during modification. For example, in the case of a matrix element having a high positive confidence level, an increase in the number of enzymes corresponding to the element increases the possibility of improving the yield of the target substance.

### (ii) S4022

The modified enzyme proposal unit 100 selects k (k in descending order of confidence level: for example, 5) modified enzymes having high confidence levels, and extracts them as modified enzymes (candidates). Then, the output device (display device) 14 outputs (displays) information on the modified enzyme (candidate) in response to an instruction from the modified enzyme proposal unit 100.

### <Details of Determination of Effectiveness of Modified Enzyme Based on Biological Experiment Results (S403)>

FIG. 8 is a flowchart illustrating details of the determination of the effectiveness of modified enzymes based on biological experiment results (S403).

### (i) S4031

The experimental data verification unit 200 acquires information on the yield (biological experiment results) when the user has modified the enzyme in the metabolic network according to the modified enzyme proposed in S4022 and performed an experiment. The biological experiment results are acquired, for example, by the user inputting the experimental result via the biological experiment result input screen 305.

It is difficult to propose how much the amount of enzyme needs to be modified. Therefore, the user needs to appropriately determine the increase/decrease amount of the enzyme. It is required to set the same modification amount (perform a biological experiment under the same conditions (the same amount and the same operation)) for each proposed enzyme (and/or enzyme determined to be modified by the user). This is because, if there is a variation in the modification amount of the enzyme, the yields cannot be appropriately compared between the modified enzymes, and the target yield becomes meaningless.

### (ii) S4032

The experimental data verification unit 200 compares the yield of the target substance due to the enzyme modification acquired in S4031 with a yield of a wild-type (comparison between the yield before modification and the yield after modification), assigns "1" if the yield increases, and assigns "0" if the yield decreases, and records information in the storage device 12 in association with the modified enzyme. Here, the wild-type means an organism having a metabolic network that operates with the amount of enzyme before modification by a biological experiment. In the case of the wild-type, yield data (yield) of the target substance is known in advance.

### <Details of Sequential Bayesian Estimation Processing (S406)>

FIG. 9 is a flowchart illustrating details of sequential Bayesian estimation processing (S406). The value of the above confidence level is affected by the probability distribution p from which each sensitivity matrix is calculated. Therefore, it is considered that the reason why the modified enzyme effective in achieving the target yield of the target substance could not be sufficiently ensured lies in the probability distribution p. Therefore, the probability distribution p is improved by the sequential Bayesian estimation processing based on the biological experiment results (the probability distribution p is changed so that the modified enzyme effective in achieving the target yield can be extracted → a value of a confidence level is not affected by the initial probability distribution).

### (i) S4061

The sequential Bayesian estimation unit 300 executes Monte Carlo sampling from a function of a product of the likelihood and the probability distribution p in order to improve the probability distribution p as described above. Specifically, Monte Carlo sampling is performed based on a function (p (x_{target substance}, _{enzyme A} = 0|θ) × p(θ)) obtained by multiplying a current probability distribution by a probability distribution for outputting a result of reducing the yield when a sampling value θ is given (that is, the matrix element (modified enzyme) to which "0" is assigned in S4032). Here, Monte Carlo sampling is to repeatedly obtain samples from any probability distribution (or a function of a constant multiple thereof). The detailed description will be omitted because Monte Carlo sampling is a well-known calculation method.

When attention is paid to one element of the sensitivity matrix, the prediction result changes depending on the sampling value of the probability distribution p. However, conceptually, it can be said that the processing of this step is processing of changing the probability distribution p so as to weight a combination of sampling values in which the yield is reduced in this way.

### (ii) S4062

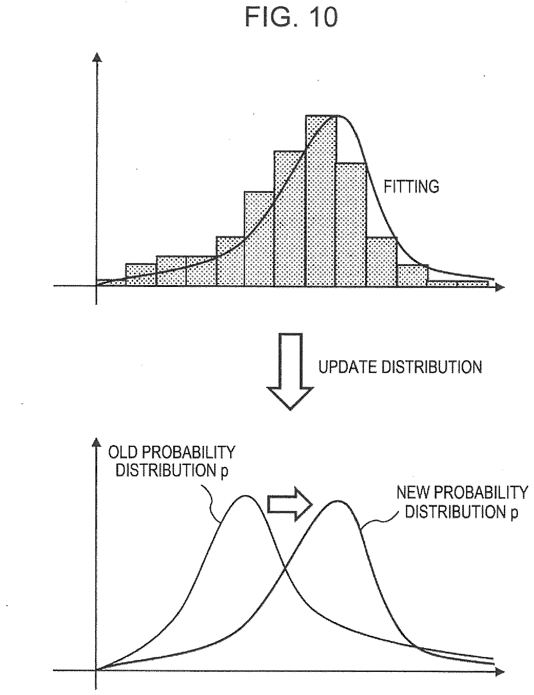

The sequential Bayesian estimation unit 300 performs fitting on the distribution of the sample obtained in S4061 to generate a new probability distribution p. As illustrated in FIG. 10, the probability distribution p is updated by fitting the multivariate log-normal distribution to the sample distribution.

When the probability distribution p is updated, the processing after the N sensitivity matrix generation loops are executed again using the new probability distribution p.

### <Summary>

(i) The present embodiment proposes metabolic network processing and method for extracting and proposing a modified enzyme that achieves a target yield of a target substance in a metabolic network. Specifically, the control device 11 reads a metabolic network processing program (a program corresponding to the flowchart in FIG. 4 and the like) from the storage device 12. The control device 11 executes, by the program, processing (S401 in FIG. 4: S4011 to S4019 in FIG. 5) of generating N sensitivity matrices using a stoichiometric matrix obtained by converting a metabolic network, a basis c of a kernel of the stoichiometric matrix, and a given probability distribution p, processing (S402 in FIG. 4: S4021 in FIG. 7) of calculating, by checking a sign of each matrix element in the N sensitivity matrices and calculating a proportion of the sign in each matrix element, a confidence level of each matrix element, and processing (S402 in FIG. 4: S4022 in FIG. 7) of extracting k (for example, five) matrix elements having high confidence levels among confidence levels for the number of matrix elements in the sensitivity matrix as modified enzymes, and processing of outputting the extracted k modified enzymes. In this way, when a modified enzyme is proposed using SSA, a modified enzyme can be always proposed by using the confidence level. As the above probability distribution p, for example, a log-normal distribution, a mixed log-normal distribution, or a gamma distribution can be applied.

When N (for example, 10000) sensitivity matrices are generated, the control device 11 (i) generates a matrix r by replacing negative matrix elements in the stoichiometric matrix with sampling values of the probability distribution (S4014 and S4015), (ii) generates a matrix r' by replacing matrix elements in the matrix r that take positive values in the stoichiometric matrix with a value of 0 (S4016), (iii) generates a matrix A by arranging the matrix r' and the basis c (S4017), and (iv) generates the sensitivity matrix by calculating an inverse matrix of the matrix A and inverting signs (S4018). The generation of the sensitivity matrix follows the structural sensitivity analysis method, and can utilize the advantage of the structural sensitivity analysis. That is, it is not required to determine a parameter from data, and the influence of enzyme modification can be predicted only from the structure of the metabolic network.

(ii) In the present embodiment, biological experiment results are utilized to verify the yield of the target substance due to the modified enzyme (candidate) once presented. Specifically, the control device 11 executes processing (S403: S4031 and S4032) of comparing a yield of the target substance obtained as a result of a biological experiment using the modified enzyme with a yield of the target substance in the metabolic network before modification (wild-type), and processing (S404 and S405) of outputting, as a final proposal, a modified enzyme by which the yield is improved as a result of the comparison processing. In this way, by considering the experimental result by giving feedback of the results of the biological experiment performed for verification of the proposal, the modified enzyme can be proposed while alleviating the dependence on the initial probability distribution p by using the confidence level. The control device 11 outputs the modified enzyme as the final proposal when a condition for the number of the modified enzymes by which the yield is improved is satisfied (for example, when there are 10 or more effective modified enzymes).

On the other hand, the control device 11 executes sequential Bayesian estimation processing and executes processing of updating the probability distribution p (S406: S4061 and S4062) when a condition for the number of the modified enzymes by which the yield is improved is not satisfied (for example, when the number of effective modified enzymes is less than 10 or when the modified enzyme (candidate) is not effective).

(iii) When the probability distribution p is updated, the control device 11 executes again a series of processing of sensitivity matrix generation → confidence level calculation → modified enzyme proposal (k) → verification based on biological experiment results → modified enzyme list output, using the updated probability distribution p. That is, the control device 11 executes processing (S4014 to S4018) of additionally generating N sensitivity matrices using the stoichiometric matrix, the basis c, and the updated probability distribution p, processing (S4021) of calculating, by checking a sign of each matrix element in the additionally generated N sensitivity matrices and calculating a proportion of the sign in each matrix element, a confidence level of each matrix element in the additionally generated N sensitivity matrices, processing (S4022) of additionally extracting k matrix elements having high confidence levels among confidence levels for the number of matrix elements in the additionally generated N sensitivity matrices as modified enzymes, and processing (S4022) of outputting the additionally extracted k modified enzymes. In this way, by repeating the feedback, the dependence on the initial probability distribution p gradually decreases, and the proposal accuracy can be improved.

(iv) Functions of the present embodiment can also be implemented by a software program code. In this case, a storage medium that records the program code is provided in a system or a device, and a computer (or CPU or MPU) of the system or the device reads the program code stored in the storage medium. In this case, the program code read from the storage medium implements the functions of the above-described embodiment, and the program code and the storage medium that stores the program code constitute the present disclosure. Examples of the storage medium for supplying such program codes include a flexible disk, a CD-ROM, a DVD-ROM, a hard disk, an optical disk, a magneto-optical disk, a CD-R, a magnetic tape, a nonvolatile memory card, and a ROM.

An operating system (OS) or the like operating on a computer may perform a part or all of actual processing based on an instruction of the program codes, and the functions of the embodiments described above may be implemented by the processing. After the program codes read from the storage medium are written in a memory on the computer, a CPU or the like of the computer may perform a part or all of actual processing based on an instruction of the program codes, and the functions of the embodiments described above may be implemented by the processing.

The software program code for implementing the functions of the embodiments may be stored, by distributing via a network, in a storage unit such as a hard disk or a memory of the system or the device or a storage medium such as a CD-RW or a CD-R, and the computer (or CPU or MPU) of the system or the device may read and execute the program code stored in the storage unit or the storage medium at the time of use.

The process and technique described here are not essentially related to any specific device and can be implemented by a combination of each component. In addition, various types of general-purpose devices can be added. A dedicated device may be constructed to execute the functions of the present embodiment. Various functions can be formed by appropriately combining a plurality of components disclosed in the present embodiment. For example, several components may be deleted from all the components shown in the embodiment, or different components may be appropriately combined.

In the present disclosure, specific embodiments are described, but these are for description (understanding of the technology of the present disclosure) without limitation in all viewpoints. Those skilled in the art will readily recognize that there are numerous combinations of hardware, software, and firmware that are suitable for implementing the techniques of the present disclosure. For example, the above-described software can be implemented in a wide range of programs or script languages such as assembler, C/C++, perl, Shell, PHP, and Java (registered trademark).

Control lines and information lines considered to be necessary for description are shown in the above-described embodiments, and not all control lines and information lines in a product are necessarily shown. All configurations may be connected to one another.

Those skilled in the art can clarify other implementations of the present disclosure from consideration of the present embodiment and each example. The description and the specific examples are merely typical, and the scope and spirit of the technology of the present disclosure are indicated by the following claims.

### Reference Signs List

10: metabolic network processing system
11: control device
12: storage device
13: input device
14: output device
301: metabolic network input screen
302: target substance input screen
303: target yield input screen
304: modified enzyme proposal screen
305: biological experiment result input screen

## Claims

1. A metabolic network processing system for extracting and proposing a modified enzyme that achieves a target yield of a target substance in a metabolic network, the metabolic network processing system comprising:
a storage device configured to hold a metabolic network processing program for extracting and outputting the modified enzyme; and
a control device configured to extract the modified enzyme by processing the metabolic network in accordance with the metabolic network processing program read from the storage device, wherein
the control device is configured to execute
processing of generating N sensitivity matrices using a stoichiometric matrix obtained by converting the metabolic network, a basis c of a kernel of the stoichiometric matrix, and a given probability distribution,
processing of calculating, by checking a sign of each matrix element in the N sensitivity matrices and calculating a proportion of the sign in each matrix element, a confidence level of each matrix element,
processing of extracting k matrix elements having high confidence levels among confidence levels for the number of matrix elements in the sensitivity matrix as modified enzymes, and
processing of outputting the extracted k modified enzymes.

2. The metabolic network processing system according to claim 1, wherein
when generating the N sensitivity matrices, the control device is configured to (i) generate a matrix r by replacing negative matrix elements in the stoichiometric matrix with sampling values of the probability distribution, (ii) generates a matrix r' by replacing matrix elements in the matrix r that take positive values in the stoichiometric matrix with a value of 0, (iii) generate a matrix A by arranging the matrix r' and the basis c, and (iv) generate the sensitivity matrix by calculating an inverse matrix of the matrix A and inverting signs.

3. The metabolic network processing system according to claim 1, wherein
the control device is configured to further execute
processing of comparing a yield of the target substance obtained as a result of a biological experiment using the modified enzyme with a yield of the target substance in the metabolic network before modification, and
processing of outputting, as a final proposal, a modified enzyme by which the yield is improved as a result of the comparison processing.

4. The metabolic network processing system according to claim 3, wherein
the control device is configured to output the modified enzyme as the final proposal when a condition for the number of the modified enzymes by which the yield is improved is satisfied.

5. The metabolic network processing system according to claim 3, wherein
the control device is configured to execute processing of updating the probability distribution by executing sequential Bayesian estimation processing when a condition for the number of the modified enzymes by which the yield is improved is not satisfied.

6. The metabolic network processing system according to claim 5, wherein
the control device is configured to execute
processing of additionally generating N sensitivity matrices using the stoichiometric matrix, the basis c, and the updated probability distribution,
processing of calculating, by checking a sign of each matrix element in the additionally generated N sensitivity matrices and calculating a proportion of the sign in each matrix element, a confidence level of each matrix element in the additionally generated N sensitivity matrices,
processing of additionally extracting k matrix elements having high confidence levels among confidence levels for the number of matrix elements in the additionally generated N sensitivity matrices as modified enzymes, and
processing of outputting the additionally extracted k modified enzymes.

7. The metabolic network processing system according to claim 1, wherein
the given probability distribution is any one of a log-normal distribution, a mixed log-normal distribution, or a gamma distribution.

8. A metabolic network processing method for extracting and proposing a modified enzyme that achieves a target yield of a target substance in a metabolic network, the metabolic network processing method comprising:
generating N sensitivity matrices using a stoichiometric matrix obtained by converting the metabolic network, a basis c of a kernel of the stoichiometric matrix, and a given probability distribution, by a control device configured to extract the modified enzyme by processing the metabolic network in accordance with a metabolic network processing program read from a storage device and extracting and outputting the modified enzyme;
calculating, by checking a sign of each matrix element in the N sensitivity matrices and calculating a proportion of the sign in each matrix element, a confidence level of each matrix element, by the control device;
extracting k matrix elements having high confidence levels among confidence levels for the number of matrix elements in the sensitivity matrix as modified enzymes, by the control device; and
outputting the extracted k modified enzymes by the control device.

9. The metabolic network processing method according to claim 8, wherein
when generating the N sensitivity matrices, the control device is configured to (i) generate a matrix r by replacing negative matrix elements in the stoichiometric matrix with sampling values of the probability distribution, (ii) generate a matrix r' by replacing matrix elements in the matrix r that take positive values in the stoichiometric matrix with a value of 0, (iii) generate a matrix A by arranging the matrix r' and the basis c, and (iv) generate the sensitivity matrix by calculating an inverse matrix of the matrix A and inverting signs.

10. The metabolic network processing method according to claim 8, further comprising:
comparing a yield of the target substance obtained as a result of a biological experiment using the modified enzyme with a yield of the target substance in the metabolic network before modification, by the control device; and
outputting, as a final proposal, a modified enzyme by which the yield is improved as a result of the comparison processing, by the control device.

11. The metabolic network processing method according to claim 10, further comprising:
outputting, by the control device, the modified enzyme as the final proposal when a condition for the number of the modified enzymes by which the yield is improved is satisfied.

12. The metabolic network processing method according to claim 10, further comprising:
updating, by the control device, the probability distribution by executing sequential Bayesian estimation processing when a condition for the number of the modified enzymes by which the yield is improved is not satisfied.

13. The metabolic network processing method according to claim 12, further comprising:
additionally generating N sensitivity matrices using the stoichiometric matrix, the basis c, and the updated probability distribution, by the control device;
calculating, by checking a sign of each matrix element in the additionally generated N sensitivity matrices and calculating a proportion of the sign in each matrix element, a confidence level of each matrix element in the additionally generated N sensitivity matrices, by the control device;
additionally extracting k matrix elements having high confidence levels among confidence levels for the number of matrix elements in the additionally generated N sensitivity matrices as modified enzymes, by the control device; and
outputting the additionally extracted k modified enzymes by the control device.

14. The metabolic network processing method according to claim 8, wherein
the given probability distribution is any one of a log-normal distribution, a mixed log-normal distribution, or a gamma distribution.
